# EUROPEAN PATENT APPLICATION

(11) **EP 4 748 324 A1**
(43) Date of publication of application: **27.05.2026**
(21) Application number: 24842498.8
(22) Date of filing: 18.07.2024
(51) Int. Cl.: A61B 17/34, A61B 1/313

(54) **NEUROSURGICAL POINTER FOR ELECTROMAGNETIC NAVIGATION**

(30) Priority: 19.07.2023 ES 202331335 U
(71) Applicant: Servicio Andaluz de Salud, 41071 Sevilla (ES)
(72) Inventor: GOMAR ALBA, Mario, 41071 Sevilla (ES); RUIZ GARCÍA, José Luis, 41071 Sevilla (ES); PLA RUIZ, José Miguel, 41071 Sevilla (ES); HUETE ALLUT, Antonio, 41071 Sevilla (ES); SAUCEDO, Leandro Ricardo, 41071 Sevilla (ES); GARCÍA PÉREZ, Fernando, 41071 Sevilla (ES); GUIL IBÁÑEZ, José Javier, 41071 Sevilla (ES)
(74) Representative: San Martin Alarcia, Esther
(86) International application number: PCT/ES2024/070461
(87) International publication number: WO 2025/017238

(57) **Abstract**

The present invention relates to a neurosurgical pointer for electromagnetic navigation, characterised in that it comprises a base from which a cylindrical rod protrudes equipped with a rounded distal end, the rod comprising an open blind longitudinal hole at the proximal end of the base, the rod being configured for insertion through the tube of the sheath of a ventriculoscope such that it protrudes from the distal end of said tube, and the open blind longitudinal hole being configured to receive the needle of a stylus of an electromagnetic navigation system, the base comprising a cavity shaped as a cylindrical portion having a circular longitudinal cross section and open toward the proximal end of said base into which a stylus body fits.

## Description

### SUBJECT OF THE INVENTION

The present invention belongs to the field of neurosurgery, and more particularly to instruments used to identify patient tissues.

The object of the present invention is a new neurosurgical pointer suitable for navigation using an electromagnetic navigation system.

### BACKGROUND OF THE INVENTION

There are four anatomical cavities in the brain, called cerebral ventricles, which are interconnected and constitute the ventricular system through which cerebrospinal fluid circulates. Certain conditions, such as hydrocephalus, require access to the cerebral ventricles. A surgical instrument called a *"ventriculoscope"* is known for this purpose.

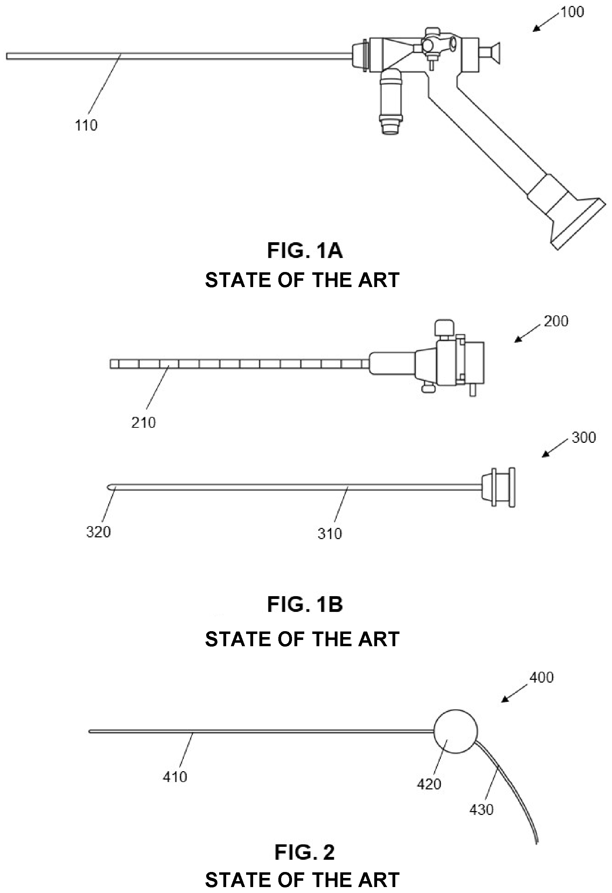

A ventriculoscope is a neurosurgical instrument designed to perform procedures in the ventricular cavities of the brain. Fig. 1A shows a ventriculoscope, in this case specifically the LOTTA model ventriculoscope manufactured by Karl Storz. As can be seen, the ventriculoscope (100) has a main tube (110) along which several conduits (not visible in the figure) extend, which are used for vision, irrigation/suction, as well as for the introduction of specific instruments for each procedure. However, the tube (110) of the ventriculoscope (100), being traversed by the aforementioned conduits, has a tip with relatively sharp edges that is not suitable for displacement between brain tissues until reaching the ventricular space.

For this reason, in order to ensure that no damage is inflicted on the patient's brain tissues during the process of introducing the ventriculoscope into the ventricular cavity, two auxiliary elements designed specifically for this purpose are known: the sheath and the trocar.

Fig. 1B shows examples of a sheath (200, above) and a trocar (300, below) that are used in combination with the ventriculoscope (100) mentioned above. The sheath (200) essentially comprises a hollow tube (210) with a diameter greater than the diameter of the tube (110) of the ventriculoscope (100). As with the tube (110) of the ventriculoscope (100), the edges of the distal end of the tube (210) of the sheath (200) are also unsuitable for insertion through brain tissues as they could cause tissue damage. The trocar (300), in turn, comprises a solid rod (310) that has a diameter smaller than the diameter of the tube (210) of the sheath (200) and a length slightly greater than the length of the tube (210) of the sheath (200). In addition, the tube (310) of the trocar (300) has a distal end (320) with an anatomical shape formed by smooth curves. When the trocar (300) is inserted into the sheath (200), the tip of the tube (310) of the trocar (300) protrudes from the distal end of the tube (210) of the sheath (200).

Sheath (200) and trocar (300) are used in combination to facilitate insertion of the tube (110) of the ventriculoscope (100) into the ventricular cavity. First, the trocar (300) is inserted into the sheath (200) until the anatomical distal end (320) of the tube (310) of said trocar (300) protrudes from the end of the sheath (200). The sheath (200) is then inserted into the patient's brain cavity until it reaches the ventricle using a suitable navigation system. Because the distal end (320) of the trocar is anatomical, tissue damage along the path is minimized. Once the sheath-trocar assembly has reached the ventricular space, the trocar (300) is removed and the tube (110) of the ventriculoscope (100) is inserted in its place. This procedure allows the ventriculoscope (100) to be inserted into the surgical field in an atraumatic manner.

Once the sheath has been navigated until its distal end is inserted into the ventricular space, it can be used not only to insert the ventriculoscope but also to insert auxiliary instruments into the ventricular cavity. Specifically, it would be desirable to have a surgical instrument that can be inserted through the sheath to mark (i.e., graze with its distal end) a certain tissue for the purpose of identifying said tissue through the images of a medical imaging system.

### DESCRIPTION OF THE INVENTION

The inventors of the present application have developed a pointer that can be introduced into the cerebral ventricle through the sheath of a ventriculoscope and that is further configured to be navigated by an electromagnetic navigation system.

Indeed, electromagnetic navigation is advantageous relative to other navigation systems, such as optical navigation, for various reasons. By way of example, it may be mentioned that the reference structures that are to be attached to the base of the navigated element make it difficult to handle. In any case, the main concept underlying electromagnetic navigation is the introduction of a known element called a stylus which, through certain electromagnetic means, is visible in a medical image by means of a system designed for this purpose.

Fig. 2 shows, by way of example, Medtronic's S8 Stealthsystem stylus. The stylus (400) essentially comprises a metal needle (410) protruding from a flat circular body (420) which, in turn, is connected by a cable (430) to certain electronic means. The electromagnetic navigation system is designed so that the needle (410) is visible in real time through a medical imaging system.

Thus, the pointer of the present invention is designed to enable electromagnetic navigation by means of a suitable stylus and can also be inserted along the sheath used for the insertion of a ventriculoscope. Specifically, the pointer of the present invention is intended to be used in combination with the sheath of a LOTTA model ventriculoscope from Karl Storz and Medtronic's S8 Stealthsystem stylus.

In short, the present invention relates to a neurosurgical pointer for electromagnetic navigation comprising a base from which a cylindrical rod with a rounded distal end protrudes.

The rod is configured for insertion through the tube of a ventriculoscope sheath such that it protrudes from the distal end of said tube. For example, according to preferred embodiments of the invention, the rod may have a diameter of between 2.4 mm and 2.6 mm and a length of between 280 mm and 300 mm. In particular, a diameter of 2.5 mm and a length of 290 mm allow the rod of the pointer of the invention to fit specifically into the sheath tube of a LOTTA model ventriculoscope from Karl Storz.

The rod further comprises a blind longitudinal hole open at a proximal end of the base. This blind longitudinal hole is configured to receive the needle of a stylus of an electromagnetic navigation system. For this purpose, the blind longitudinal hole may have a diameter of between 1.8 mm and 2.2 mm and a length of between 260 mm and 280 mm. Particularly preferably, a diameter of 2 mm and a length of 270 mm allow the Medtronic S8 Stealth stylus to be specifically inserted into the hole.

The base further comprises a cavity in the shape of a cylindrical portion with a circular longitudinal section and open towards the proximal end of said base, so that a body of the stylus fits into the cavity. The cavity may have a radius of between 124 mm and 126 mm, with 125 mm being a particularly preferred radius, as it allows the body of a Medtronic S8 Stealth stylus to be specifically inserted. The thickness of the base may be between 11 mm and 13 mm, preferably 12 mm. The stem and hole lengths mentioned above do not include the base.

In principle, the pointer of the invention can be made of different materials, such as stainless steel or similar. However, in a particularly preferred embodiment of the invention, the pointer is manufactured using 3D printing. In that case, the material from which it is made is polypropylene.

### BRIEF DESCRIPTION OF THE DRAWINGS

FIG. 1A shows an example of a ventriculoscope according to the prior art, specifically a LOTTA model ventriculoscope from Karl Storz.
FIG. 1B shows a sheath (top) and a trocar (bottom) used for the introduction of the ventriculoscope tube of Fig. 1A.
FIG. 2 shows a stylus of an electromagnetic navigation system according to the prior art, specifically the Medtronic S8 Stealthsystem stylus.
FIG. 3 shows a perspective view of the neurosurgical pointer according to the present invention.
FIG. 4 shows a longitudinal section of the neurosurgical pointer according to the present invention.
FIG. 5 shows a perspective view of the pointer of the invention with a surgical navigation stylus partially inserted into its longitudinal hole.

### PREFERRED EMBODIMENT OF THE INVENTION

A particular example of a neurosurgical pointer (1) according to the present invention is described below with reference to the figures.

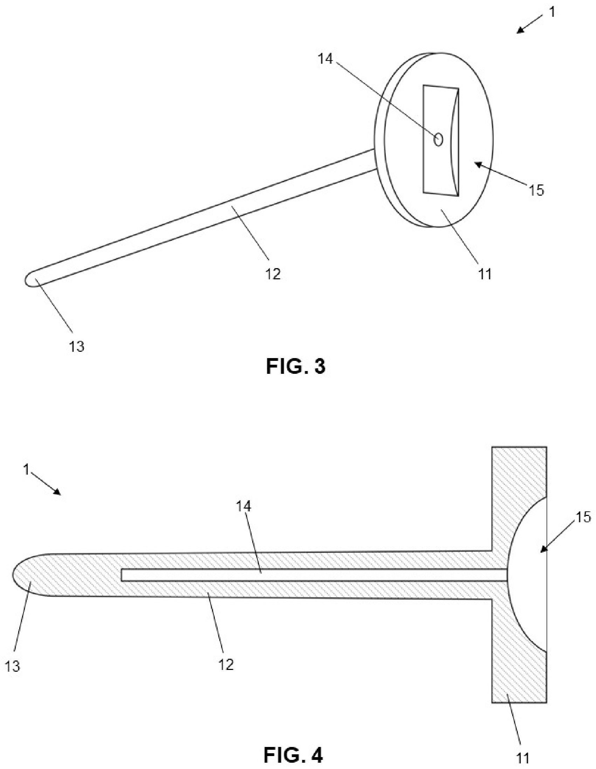

Figs. 3 and 4 respectively show a perspective view of the pointer (1) showing its different parts. The pointer (1) has a proximal base (11) from which a rod (12) emerges in a distal direction. The rod (12) has a cylindrical shape, with a length of 290 mm and a diameter of 2.5 mm, which allows it to be inserted through the tube (210) of the sheath (200) of a LOTTA ventricular from Karl Storz as shown in Fig. 1B. When fully inserted, the distal end (13) of the rod (12) protrudes from the tip of the tube (210). For this reason, to avoid causing damage to the tissues when marking them, the distal end (13) of the rod (12) has a rounded shape.

The base (11), which is 12 mm thick, has a diameter substantially larger than the rod (12), so that it abuts the proximal end of the tube (210) of the sheath (200). In addition, the base (11) comprises a cavity (15) at its proximal end for housing the body (420) of a stylus (400) to be used in combination with the pointer (1) of the invention. Specifically, the cavity (15) has, in this example, the shape of a portion of a cylinder with a longitudinal section in the form of a circular arc with a diameter of 125 mm. This will allow the body (420) of a Medtronic S8 Stealth stylus, which has the shape of a circular tablet with a slightly smaller diameter, to fit specifically inside it. Fitting the body (420) of the stylus (400) into the cavity (15) allows one element to be fixed more precisely to the other, while also preventing relative rotation between the two.

The rod (12) of the pointer (1) of the invention also has a blind longitudinal hole (14) running through it. This longitudinal hole (14) is open towards the proximal end of the pointer (1), i.e., the proximal mouth of the hole (14) is inside the cavity (15) of the base (11) of the pointer (1). The distal end of the hole (14), in turn, is blind and is located at some point along the length of the rod (12). Specifically, the blind longitudinal hole (14) has a length of 270 mm and a thickness of 2 mm. The distance between the distal end of the hole (14) and the distal end (13) of the rod (12) is therefore approximately 20 mm.

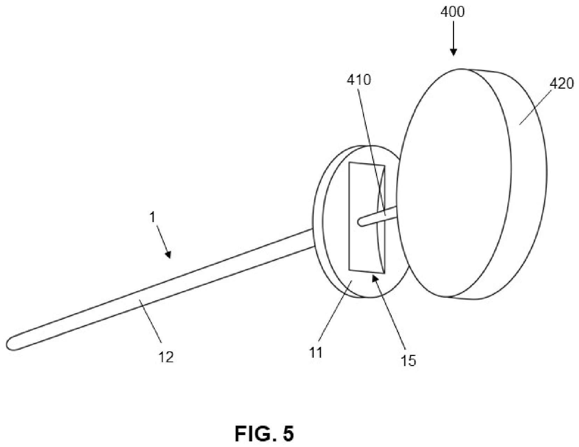

This configuration allows the pointer (1) of the invention to be used to mark tissues located inside a patient's ventricular cavity. To do this, the needle (410) of an electromagnetic navigation stylus (400) is first inserted into the blind longitudinal hole (14) of the pointer (1), as shown in Fig. 5. The needle (410) is inserted all the way in, leaving the body (420) of the stylus (400) fitted inside the cavity (15) of the base (11) of the pointer (1).

Once this has been done, the functions of the electromagnetic navigation system are used to draw an imaginary straight line between the end of the needle (410) of the stylus (400), which is located at an intermediate point on the rod (12), specifically at 20 mm from the end of its distal end (13) of said rod (12). To that end, known references are taken in a manner known in the art.

Next, once the sheath (200) has been inserted into the patient's ventricular cavity using the procedure described above in this document, the rod (12) of the pointer (1) of the invention is inserted through the tube (210) of said sheath (200). The rod (12) is inserted until its distal end (13) protrudes through the tip of said tube (210), thus remaining inside the ventricular cavity.

Once the previous steps have been carried out, the medical professional is able to mark the tissues with the pointer (1), viewing the position of the distal end (13) of the pointer (1) through medical images in real time, thereby facilitating the identification of the tissues.

## Claims

1. Neurosurgical pointer (1) for electromagnetic navigation, **characterized in that** it comprises a base (11) from which a cylindrical rod (12) protrudes, equipped with a rounded distal end (13), the rod (12) comprising a blind longitudinal hole (14) open at a proximal end of the base (11),
wherein the rod (12) is configured for insertion through the tube (210) of the sheath (200) of a ventriculoscope (100) such that it protrudes from the distal end of said tube (210), and
wherein the blind longitudinal hole (14) is configured to receive the needle (410) of a stylus (400) of an electromagnetic navigation system, the base (11) comprising a cavity (15) in the shape of a cylindrical portion with a circular longitudinal section and open towards the proximal end of said base (11) in which a body (420) of the stylus (400) fits.

2. Pointer (1) according to claim 1, wherein the rod (12) has a diameter of between 2.4 mm and 2.6 mm.

3. Pointer (1) according to claim 2, wherein the rod (12) has a diameter of 2.5 mm.

4. Pointer (1) according to any of the preceding claims, wherein the rod (12) has a length of between 280 mm and 300 mm.

5. Pointer (1) according to claim 4, wherein the rod (12) has a length of 290 mm.

6. Pointer (1) according to any of the preceding claims, wherein the blind longitudinal hole (14) has a diameter of between 1.8 mm and 2.2 mm.

7. Pointer (1) according to claim 6, wherein the blind longitudinal hole (14) has a diameter of 2.0 mm.

8. Pointer (1) according to any of the preceding claims, wherein the blind longitudinal hole (14) has a length of between 260 mm and 280 mm.

9. Pointer (1) according to claim 8, wherein the blind longitudinal hole (14) has a length of 270 mm.

10. Pointer (1) according to any of the preceding claims, wherein the cavity (15) at the proximal end of the base (11) has a radius of between 124 mm and 126 mm.

11. Pointer (1) according to claim 10, wherein the cavity (15) at the proximal end of the base (11) has a radius of 125 mm.

12. Pointer (1) according to any of the preceding claims, which is manufactured by 3D printing.
